# EUROPEAN PATENT SPECIFICATION

(11) **EP 4 403 546 B1**
(45) Date of publication and mention of the grant of the patent: **04.03.2026**
(21) Application number: 22867257.2
(22) Date of filing: 30.08.2022
(51) Int. Cl.: C07C 45/68, C07C 49/323

(54) **METHOD FOR PRODUCING 1,3-DISUBSTITUTED BICYCLO[1.1.1]PENTANE BY PHOTOREACTION**
VERFAHREN ZUR HERSTELLUNG VON 1,3-DISUBSTITUIERTEM BICYCLO[1.1.1!PENTAN DURCH PHOTOREAKTION
PROCÉDÉ DE PRODUCTION DE BICYCLO[1.1.1]PENTANE 1,3-DISUBSTITUÉ PAR PHOTORÉACTION

(30) Priority: 13.09.2021 JP 2021148842
(43) Date of publication of application: 24.07.2024
(73) Proprietor: FUJIFILM Corporation, Tokyo 106-8620 (JP)
(72) Inventor: TSUNA, Kazuhiro, Ashigarakami-gun, Kanagawa 258-8577 (JP); IWASAKI, Koichi, Ashigarakami-gun, Kanagawa 258-8577 (JP); WADA, Kenji, Ashigarakami-gun, Kanagawa 258-8577 (JP)
(74) Representative: HGF
(86) International application number: PCT/JP2022/032638
(87) International publication number: WO 2023/037935

(56) References cited:
- WO-A1-2020/248126
- CN-A- 105 294 442
- CN-A- 105 294 442
- JP-A- 2007 022 921
- JP-A- 2007 022 921
- JP-A- 2019 510 012
- US-A1- 2021 061 730

## Description

### BACKGROUND OF THE INVENTION

### 1. Field of the Invention

The present invention relates to a method for producing 1,3-disubstituted bicyclo[1.1.1]pentane by a photoreaction.

### 2. Description of the Related Art

A bicyclo[1.1.1]pentane (BCP) compound has attracted attention as a biologically active compound. A BCP motif has high three-dimensionality and high biological safety while having biological functionality equivalent to a para-substituted phenyl group, an alkynyl group, a tert-butyl group, and the like, so that it is expected to be applied to drug delivery. A 1,3-disubstituted form of BCP is actually introduced as a building block in synthesis of a pharmaceutical drug candidate compound.

A method of subjecting [1.1.1]propellane and a 1,2-diketone compound to a photoreaction to obtain the 1,3-disubstituted form of BCP has been known. For example, as a method of synthesizing 1,3-diacetyl BCP, JP2020-533330A discloses a method of subjecting [1.1.1]propellane and 2,3-butanedione to a photoreaction. In addition, JP2019-510012A discloses that [1.1.1]propellane and a glyoxylic acid compound are subjected to a photoreaction to obtain a 1,3-disubstituted form of BCP.

### SUMMARY OF THE INVENTION

An object of the present invention is to provide a method for producing 1,3-disubstituted BCP, in which, in a case where [1.1.1]propellane and a 1,2-diketone compound are subjected to a photoreaction to obtain 1,3-disubstituted BCP, the photoreaction can be promoted and a target 1,3-disubstituted BCP can be obtained with high efficiency.

The object of the present invention has been achieved by the following methods.
[1] A method for producing 1,3-disubstituted bicyclo[1.1.1]pentane, the method comprising:
   subjecting [1.1.1]propellane and a 1,2-diketone compound to a photoreaction in a solvent including an acyclic ether solvent having 5 or more carbon atoms to obtain 1,3-disubstituted bicyclo[1.1.1]pentane,
   in which a reaction solution coexists with a cyclic ether compound, wherein the 1,2-diketone compound is a compound having a "*-C(=O)-C(=O)-*" structure, wherein * represents a linking site; wherein the content of the cyclic ether compound in the reaction solution is 10% to 50% by mass.
[2] The method for producing 1,3-disubstituted bicyclo[1.1.1]pentane according to [1],
   in which the acyclic ether solvent has 5 to 10 carbon atoms.
[3] The method for producing 1,3-disubstituted bicyclo[1.1.1]pentane according to [1] or [2],
   in which the acyclic ether solvent has 5 or 6 carbon atoms.
[4] The method for producing 1,3-disubstituted bicyclo[1.1.1]pentane according to any one of [1] to [3],
   in which the 1,2-diketone compound is a diacetyl compound or a glyoxylic acid compound.
[5] The method for producing 1,3-disubstituted bicyclo[1.1.1]pentane according to any one of [1] to [4],
   in which the cyclic ether compound is a tetrahydrofuran compound.
[6] The method for producing 1,3-disubstituted bicyclo[1.1.1]pentane according to any one of [1] to [5],
   in which the photoreaction is carried out by irradiation with light having a wavelength of 200 to 600 nm.
[7] The method for producing 1,3-disubstituted bicyclo[1.1.1]pentane according to any one of [1] to [6],
   in which a reaction temperature of the photoreaction is set to -50°C to 50°C.

In the present invention or the specification, any numerical range expressed using "to" refers to a range including the numerical values before and after the "to" as a lower limit value and an upper limit value, respectively.

With the method for producing 1,3-disubstituted BCP according to the aspect of the present invention, it is possible to promote a photoreaction of [1.1.1]propellane and a 1,2-diketone compound, and it is possible to obtain a target 1,3-disubstituted BCP by the photoreaction with high efficiency.

### BRIEF DESCRIPTION OF THE DRAWINGS

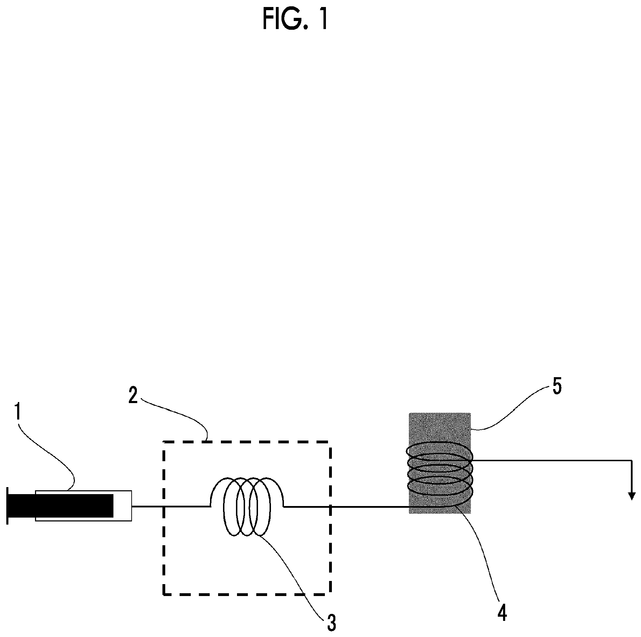

Fig. 1 is a schematic diagram of a flow-type reaction system used in Examples.

### DESCRIPTION OF THE PREFERRED EMBODIMENTS

In the method for producing 1,3-disubstituted BCP according to the embodiment of the present invention (hereinafter, also referred to as a production method according to the embodiment of the present invention), [1.1.1]propellane and a 1,2-diketone compound are subjected to a photoreaction in a solvent including an acyclic ether solvent having 5 or more carbon atoms. The production method according to the embodiment of the present invention is a method in which a reaction solution in the photoreaction coexists with a cyclic ether compound, and as a result, a target 1,3-disubstituted BCP can be obtained with high efficiency. In the reaction system, the cyclic ether functions as an additive for promoting the reaction.

### [Acyclic ether solvent having 5 or more carbon atoms]

The number of carbon atoms in the acyclic ether solvent having 5 or more carbon atoms, which is used in the production method according to the embodiment of the present invention, is preferably 5 to 10, more preferably 5 to 8, still more preferably 5 to 7, and even more preferably 5 or 6.

By using the acyclic ether solvent having 5 or more carbon atoms, it is possible to effectively exhibit the reaction promoting action by the addition of the cyclic ether.

It is more preferable that the acyclic ether solvent having 5 or more carbon atoms is at least one of cyclopentylmethyl ether or methyl tert-butyl ether.

The solvent used in the production method according to the embodiment of the present invention may include a solvent other than the acyclic ether having 5 or more carbon atoms (solvent other than "acyclic ether having 5 or more carbon atoms") (excluding the cyclic ether compound). The solvent other than the acyclic ether solvent having 5 or more carbon atoms is not particularly limited, and can be appropriately used as long as the effects of the present invention are not impaired. Preferred examples of the solvent other than the acyclic ether solvent having 5 or more carbon atoms include hydrocarbon solvents, and among these, an aliphatic hydrocarbon solvent (for example, hexane, cyclohexane, pentane, heptane, and the like) is preferable. In a case of including the acyclic ether having 5 or more carbon atoms and other solvents, as an amount ratio thereof, in terms of mass ratio, [Acyclic ether solvent having 5 or more carbon atoms]/[Solvent other than acyclic ether solvent having 5 or more carbon atoms] is preferably 1/10 or more, more preferably 1/5 or more, still more preferably 1/2 or more, and even more preferably 1/1 or more. That is, a proportion of the acyclic ether solvent having 5 or more carbon atoms in the solvent is preferably 50% by mass or more, 60% by mass or more, or 65% by mass or more.

### [[1.1.1]propellane]

The [1.1.1]propellane can be generally obtained by reacting 1,1-dibromo-2,2-bis(chloromethyl)cyclopropane with an organometallic reagent.

As the organometallic compound, alkyllithium, aryllithium, or the like can be widely used.

The reaction itself of reacting 1,1-dibromo-2,2-bis(chloromethyl)cyclopropane with an organometallic reagent to obtain [1.1.1]propellane is already known, and for example, Chem. Commun., 2021, vol. 57, p. 2871 to 2874 can be referred to.

### [1,2-Diketone compound]

The 1,2-diketone compound is a compound having a "*-C(=O)-C(=O)-*" structure. * represents a linking site. The 1,2-diketone compound is easily cleaved in two carbonyl groups by light energy, and the 1,2-diketone compound undergoes a photoreaction with the [1.1.1]propellane to produce 1,3-disubstituted BCP.

Examples of the 1,2-diketone compound include a diacetyl compound (2,3-butanedione compound) and a glyoxylic acid compound. In the present invention, the term "... compound" means "compound having ... skeleton". For example, "diacetyl compound" includes not only diacetyl itself (2,3-butanedione), but also a form in which at least a part of hydrogen atoms in the diacetyl is substituted. The form in which at least a part of hydrogen atoms in the diacetyl is substituted includes a form in which a structure is changed to a structure having an unsaturated bond, such as changing -CH₃ of diacetyl to -CH=CH₂, -CN, or the like. In addition, a form in which two or three hydrogen atoms of -CH₃ of diacetyl are substituted by substituents includes a form in which two or three substituents are linked to each other to form a ring.

Preferred specific examples of the above-described diacetyl compound are shown below. In the table below, D indicates deuterium.

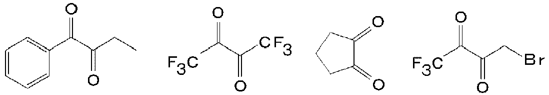

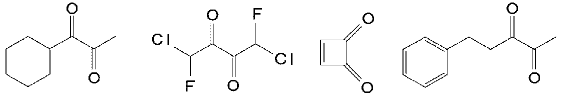

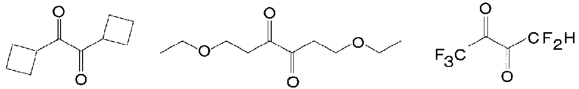

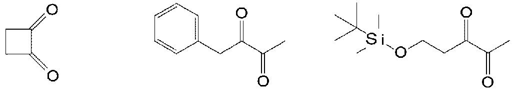

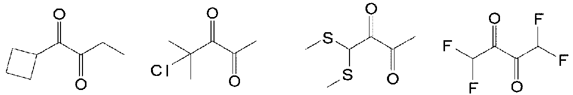

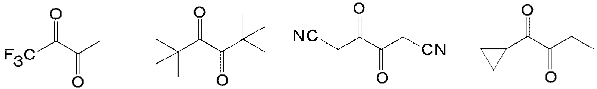

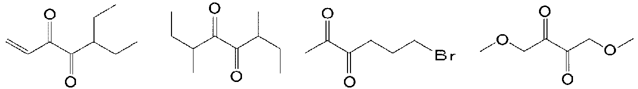

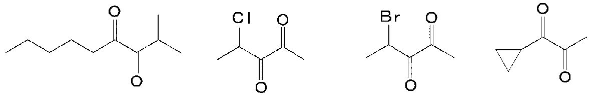

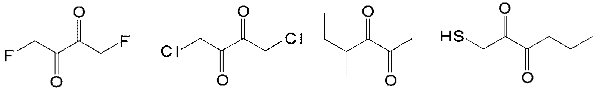

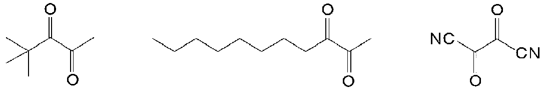

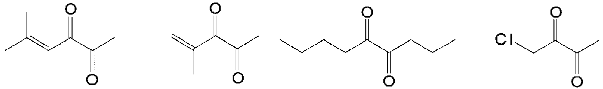

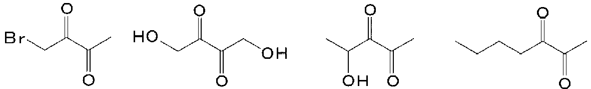

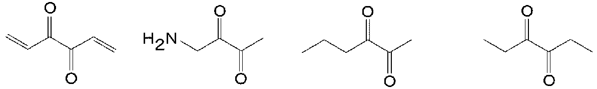

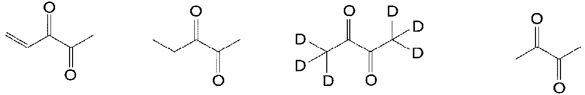

Preferred specific examples of the above-described glyoxylic acid compound are shown below.

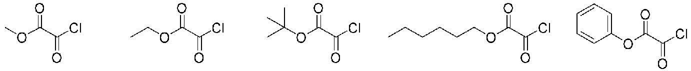

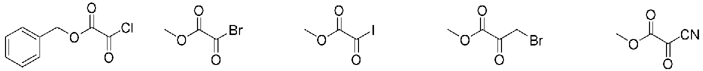

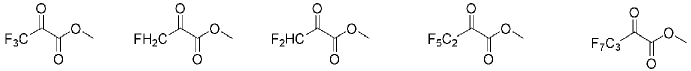

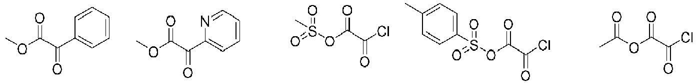

### [Cyclic ether compound]

In the present invention, the cyclic ether compound does not act as a solvent, and is added in a small amount to function as a reaction promoter. That is, in the production method according to the embodiment of the present invention, the acyclic ether solvent having 5 or more carbon atoms is used as the solvent, and the cyclic ether compound is blended as an additive different from the solvent. By the addition of the cyclic ether compound, it is possible to significantly improve a reaction rate of the photoreaction specified in the present invention, and it is possible to effectively increase the yield of the target 1,3-disubstituted BCP. The reason for this is not clear, but it is presumed that active species in which two carbonyl groups of the 1,2-diketone compound are cleaved by light energy are stabilized by the cyclic ether compound, and re-bonding between the active species is suppressed. As shown in Examples described later, no reaction promoting action is observed even in a case where various compounds other than the cyclic ether compound are added. That is, in the photoreaction specified in the present invention, the reaction promoting action of the cyclic ether compound is unique to the cyclic ether compound, in which the reaction promoting action is not recognized in other compounds.

Preferred examples of the cyclic ether compound include tetrahydrofuran (THF) compounds (for example, tetrahydrofuran (THF), 2-methyltetrahydrofuran (2-methyl THF), 2,5-dihydrofuran, tetrahydrofuran-d8 (deuterated tetrahydrofuran), 3-methyltetrahydrofuran, 2-hydroxytetrahydrofuran, 2-chlorotetrahydrofuran, and the like), tetrahydropyrane, 4-methyltetrahydropyrane, furan, and 1,4-dioxane. Among these, a THF compound is preferable, and at least one of THF or 2-methyl THF is more preferable.

In the photoreaction, a content of the cyclic ether compound in a reaction solution (solution subjected to the photoreaction; also referred to as a reaction substrate solution) is 10% to 50% by mass, more preferably 10% to 25% by mass, further more preferably 12 to 23, and even further more preferably 15% to 22% by mass.

### [Photoreaction]

In the present invention, the photoreaction is a reaction in which [1.1.1]propellane and the 1,2-diketone compound are reacted by irradiation with light to produce the 1,3-disubstituted BCP. The reaction scheme is as follows. R represents a hydrogen atom or a substituent.

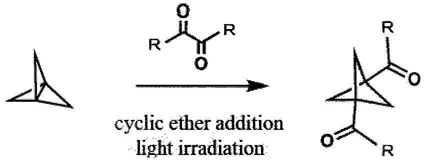

In the present invention, the photoreaction is carried out in a solvent including the acyclic ether solvent having 5 or more carbon atoms, by adding the cyclic ether compound as a reaction promoter as described above.

The photoreaction is preferably carried out by irradiation with light having a wavelength of 300 to 500 nm, more preferably carried out by irradiation with light having a wavelength of 320 to 450 nm, and still more preferably carried out by irradiation with light having a wavelength of 340 to 430 nm. As a light source, a light emitting diode (LED) lamp, an ultraviolet (UV) lamp, an incandescent lamp (for example, a tungsten lamp), or the like can be used without particular limitation.

The photoreaction may be carried out by a batch-type reaction or a flow-type reaction. From the viewpoint of light absorption efficiency, it is preferable to carry out a flow-type reaction.

The photoreaction in the flow-type reaction can be carried out using a photoreactor. The photoreactor can be constructed, for example, by winding a light-transmitting reaction pipe (tube) around a light source cooling pipe. A light source is disposed in the light source cooling pipe and is cooled by water or another solvent. It is also preferable that the photoreactor is covered with a reflective material such as aluminum foil in order to suppress light loss. Examples of a material of the light-transmitting reaction pipe include a tetrafluoroethylene-hexafluoropropylene copolymer (FEP) tube, a tetrafluoroethylene-perfluoroalkyl vinyl ether copolymer (PFA) tube, and a polytetrafluoroethylene (PTFE) tube, but it is not limited thereto and various materials can be used as long as the effects of the present invention are not impaired.

The flow-type reaction is carried out, for example, by an aspect that a solution in which [1.1.1]propellane, the 1,2-diketone compound, and the cyclic ether compound are present together in a solvent including the acyclic ether solvent having 5 or more carbon atoms is prepared, and this solution is pumped into the reaction pipe and allowed to flow through the reaction pipe.

In addition, it is also carried out by an aspect that a solution obtained by dissolving [1.1.1]propellane in a solvent including the acyclic ether solvent having 5 or more carbon atoms and a solution obtained by dissolving the 1,2-diketone compound in a solvent including the acyclic ether solvent having 5 or more carbon atoms are passed through different flow channels and then combined, and a photoreaction occurs in a photoreactor while the combined solution is allowed to flow downstream. In the reaction, the cyclic ether compound as a reaction promoter is added at the latest before the photoreaction. For example, the cyclic ether compound may be contained in the [1.1.1]propellane solution or may be contained in the 1,2-diketone compound solution, or a solution containing cyclic ether may be combined before the photoreaction.

Although the preferred aspects of the reaction mode have been described above, the present invention is not limited to the above-described aspects except as specified in the present invention. For example, application of the batch-type reaction is also a preferred embodiment of the present invention.

Subsequently, preferred reaction conditions of the photoreaction will be described.

A reaction temperature of the photoreaction can be set, for example, to -50°C to 50°C, and from the viewpoint of improving the yield, it is preferably set to -30°C to 30°C, more preferably set to -20°C to 20°C, and still more preferably set to -10°C to 10°C.

In addition, a reaction time of the photoreaction is not particularly limited, and can be appropriately set. For example, the reaction time can be 0.001 to 7000 minutes, and it may be 0.01 to 5,000 minutes or may be 0.1 to 700 minutes.

In the above-described reaction, a reaction molar ratio of [1.1.1]propellane to the 1,2-diketone compound is appropriately adjusted in consideration of stoichiometry and the like. For example, [[1.1.1]propellane]/[1,2-diketone compound] can be set to 1/0.5 to 1/10, preferably 1/1 to 1/5 and more preferably 1/1 to 1/2.

A concentration of each of the [1.1.1]propellane and the 1,2-diketone compound in the reaction solution (solution subjected to the photoreaction) is appropriately set according to the purpose and in consideration of the above-described reaction molar ratio. For example, the concentration of the [1.1.1]propellane in the reaction solution can be set to 0.01 to 3 mol/L, and it is also preferably set to 0.1 to 0.5 mol/L.

The yield in the production method according to the embodiment of the present invention (the yield of the 1,3-disubstituted BCP in the reaction of obtaining 1,3-disubstituted BCP by the photoreaction of [1.1.1]propellane and the 1,2-diketone compound, that is, [Molar ratio of produced 1,3-disubstituted BCP]/[Molar ratio of [1.1.1]propellane as starting raw material]) is preferably 80% or more, more preferably 85% or more, still more preferably 90% or more, and even more preferably 92% or more. Such a high yield can be achieved by adding the cyclic ether compound as a reaction promoter.

In the production method according to the embodiment of the present invention, the 1,3-disubstituted BCP produced by the photoreaction can be separated and purified. As this separation or purification method, a general method can be appropriately applied. For example, column chromatography, recrystallization, reprecipitation, sublimation, or the like can be applied alone or in combination.

Hereinafter, the present invention will be more specifically described based on Examples, but the present invention is not limited to Examples.

### Examples

### [Example 1]

1,3-diacetyl BCP was obtained according to the following scheme.

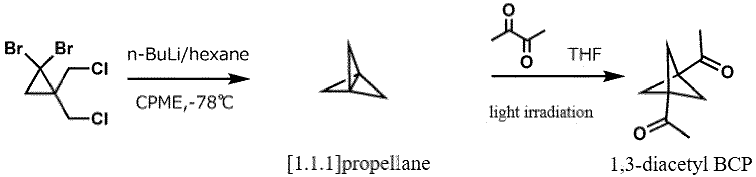

### <Preparation of [1.1.1]propellane>

100 mL of cyclopentyl methyl ether (CPME) and 10 g of 1,1-dibromo-2,2-bis(chloromethyl)cyclopropane were charged into a 500 mL three-neck flask, and cooled to -78°C in a nitrogen atmosphere. 42 mL of a solution obtained by dissolving n-butyllithium in n-hexane at a concentration of 1.6 M was added thereto, and the mixture was stirred for 10 minutes for reaction. Thereafter, the mixture was heated to 0°C and further stirred for 30 minutes. In this way, [1.1.1]propellane in the solvent was obtained. 30 mL of water was added thereto, the temperature was raised to room temperature (25°C), and then liquid separation was performed to obtain an organic layer. The organic layer was cooled to 0°C, a distillation device was connected, a receiving flask was cooled to -78°C to carry out a distillation treatment, and the [1.1.1]propellane was transferred to the receiving flask. As a result, a CPME/n-hexane (3.6/1, mass ratio) solution containing [1.1.1]propellane at a concentration of 0.15 M was obtained. The yield of [1.1.1]propellane was 62%. From the following data, it was confirmed that the [1.1.1]propellane was obtained.

Chemical shift σ (ppm) in ¹H-NMR (400 MHz, solvent: DMSO-d6, internal reference substance: tetramethylsilane (TMS)) = 2.04 (6H, s)

### <Preparation of 1,3-diacetyl BCP>

1,3-diacetyl BCP was prepared by a flow-type reaction. An outline of the used flow-type reaction system is as shown in Fig. 1. In the flow-type reaction system, a stainless steel (SUS 316) tube 3 having an inner diameter of 1.0 mm and a length of 1.0 m was disposed in a constant-temperature tank 2 set to 0°C, and a PFA tube 4 having an inner diameter of 1.6 mm and a length of 3.0 m was connected downstream thereof. The PFA tube 4 was wound around a light source cooling pipe 5. As a light source in the light source cooling pipe 5, an LED (100 W) lamp having a wavelength of 385 nm was disposed. In addition, the temperature of the light source cooling pipe 5 (corresponding to a reaction temperature of a photoreaction) was set to 0°C. A reaction substrate solution 1 was fed by a syringe pump.

The CPME/n-hexane solution containing [1.1.1]propellane at a concentration of 0.15 M, which had been obtained in <Preparation of [1.1.1]propellane> described above, was mixed with 2,3-butanedione having 2.0 molar equivalent relative to [1.1.1]propellane (twice the molar amount of [1.1.1]propellane), and THF was added thereto and mixed such that a concentration shown in the table below was set. The mixed solution was cooled to 5°C, and nitrogen bubbling was carried out for 5 minutes to prepare a reaction substrate solution (a reaction solution before being subjected to a photoreaction). The reaction substrate solution was fed into a flow channel of the flow-type reaction system at a flow rate of 1.2 mL/min, and 1,3-diacetyl BCP was produced by a photoreaction. The reaction time (flow time in the PFA tube wound around the light source cooling pipe 5) of the photoreaction was 5 minutes. Using the obtained solution after the reaction, yield of 1,3-diacetyl BCP obtained by the photoreaction of [1.1.1]propellane and 2,3-butanedione was calculated by a nuclear magnetic resonance method (internal standard: biphenyl). The result is shown in the table below. From the following data, it was confirmed that 1,3-diacetyl BCP [another name: 1,1'-(bicyclo[1.1.1]pentane-1,3-diyl)bis(ethan-1-one)] was obtained.

Chemical shift σ (ppm) in ¹H-NMR (400 MHz, solvent: DMSO-d6, internal reference substance: tetramethylsilane (TMS)) = 2.16 (6H, s), 2.10 (6H, s)

### [Examples 2 to 4]

1,3-diacetyl BCP was obtained in the same manner as in Example 1, except that, in Example 1, the THF concentration in the reaction substrate solution was changed as shown in the table below. The yield of 1,3-diacetyl BCP obtained by the photoreaction of [1.1.1]propellane and 2,3-butanedione is shown in the table below.

### [Example 5]

An MTBE/n-hexane solution containing [1.1.1]propellane at a concentration of 0.15 M was obtained in the same manner as <Preparation of [1.1.1]propellane> in Example 1, except that, in Example 1, CPME used in <Preparation of [1.1.1]propellane> was changed to methyl tert-butyl ether (MTBE).

Thereafter, 1,3-diacetyl BCP was obtained in the same manner as <Preparation of 1,3-diacetyl BCP> in Example 1, except that the above-described MTBE/n-hexane solution containing [1.1.1]propellane at a concentration of 0.15 M was used instead of the CPME/n-hexane solution containing [1.1.1]propellane at a concentration of 0.15 M. The yield of 1,3-diacetyl BCP obtained by the photoreaction of [1.1.1]propellane and 2,3-butanedione is shown in the table below.

### [Examples 6 to 8]

1,3-diacetyl BCP was obtained in the same manner as in Example 5, except that, in Example 5, the THF concentration in the reaction substrate solution was changed as shown in the table below. The yield of 1,3-diacetyl BCP obtained by the photoreaction of [1.1.1]propellane and 2,3-butanedione is shown in the table below.

### [Example 9]

1,3-diacetyl BCP was obtained in the same manner as in Example 1, except that, in Example 1, 2-methyl THF was used instead of THF. The yield of 1,3-diacetyl BCP obtained by the photoreaction of [1.1.1]propellane and 2,3-butanedione is shown in the table below.

### [Examples 10 to 12]

1,3-diacetyl BCP was obtained in the same manner as in Example 9, except that, in Example 9, the 2-methyl THF concentration in the reaction substrate solution was changed as shown in the table below. The yield of 1,3-diacetyl BCP obtained by the photoreaction of [1.1.1]propellane and 2,3-butanedione is shown in the table below.

### [Example 13]

1,3-diacetyl BCP was obtained in the same manner as in Example 9, except that, in Example 9, the MTBE/n-hexane solution containing [1.1.1]propellane at a concentration of 0.15 M, which was the same as in Example 5, was used instead of the CPME/n-hexane solution containing [1.1.1]propellane at a concentration of 0.15 M. The yield of 1,3-diacetyl BCP obtained by the photoreaction of [1.1.1]propellane and 2,3-butanedione is shown in the table below.

### [Examples 14 to 16]

1,3-diacetyl BCP was obtained in the same manner as in Example 13, except that, in Example 13, the 2-methyl THF concentration in the reaction substrate solution was changed as shown in the table below. The yield of 1,3-diacetyl BCP obtained by the photoreaction of [1.1.1]propellane and 2,3-butanedione is shown in the table below.

### [Example 17]

1,3-diacetyl BCP was obtained in the same manner as in Example 1, except that, as the light source provided in the light source cooling pipe of the flow-type reaction system, an LED (100 W) lamp having a wavelength of 405 nm was provided instead of the LED (100 W) lamp having a wavelength of 385 nm. The yield of 1,3-diacetyl BCP obtained by the photoreaction of [1.1.1]propellane and 2,3-butanedione is shown in the table below.

### [Example 18]

1,3-diacetyl BCP was obtained in the same manner as in Example 17, except that, in Example 17, the THF concentration in the reaction substrate solution was changed as shown in the table below. The yield of 1,3-diacetyl BCP obtained by the photoreaction of [1.1.1]propellane and 2,3-butanedione is shown in the table below.

### [Example 19]

1,3-diacetyl BCP was obtained in the same manner as in Example 17, except that, in Example 17, the MTBE/hexane solution containing [1.1.1]propellane at a concentration of 0.15 M, which was the same as in Example 5, was used instead of the CPME/hexane solution containing [1.1.1]propellane at a concentration of 0.15 M. The yield of 1,3-diacetyl BCP obtained by the photoreaction of [1.1.1]propellane and 2,3-butanedione is shown in the table below.

### [Example 20]

1,3-diacetyl BCP was obtained in the same manner as in Example 19, except that, in Example 19, the THF concentration in the reaction substrate solution was changed as shown in the table below. The yield of 1,3-diacetyl BCP obtained by the photoreaction of [1.1.1]propellane and 2,3-butanedione is shown in the table below.

### [Example 21]

1,3-diacetyl BCP was obtained in the same manner as in Example 1, except that, as the light source provided in the light source cooling pipe of the flow-type reaction system, an LED (100 W) lamp having a wavelength of 365 nm was provided instead of the LED (100 W) lamp having a wavelength of 385 nm. The yield of 1,3-diacetyl BCP obtained by the photoreaction of [1.1.1]propellane and 2,3-butanedione is shown in the table below.

### [Example 22]

1,3-diacetyl BCP was obtained in the same manner as in Example 21, except that, in Example 21, the THF concentration in the reaction substrate solution was changed as shown in the table below. The yield of 1,3-diacetyl BCP obtained by the photoreaction of [1.1.1]propellane and 2,3-butanedione is shown in the table below.

### [Example 23]

1,3-diacetyl BCP was obtained in the same manner as in Example 21, except that, in Example 21, the MTBE/hexane solution containing [1.1.1]propellane at a concentration of 0.15 M, which was the same as in Example 5, was used instead of the CPME/hexane solution containing [1.1.1]propellane at a concentration of 0.15 M. The yield of 1,3-diacetyl BCP obtained by the photoreaction of [1.1.1]propellane and 2,3-butanedione is shown in the table below.

### [Example 24]

1,3-diacetyl BCP was obtained in the same manner as in Example 23, except that, in Example 23, the THF concentration in the reaction substrate solution was changed as shown in the table below. The yield of 1,3-diacetyl BCP obtained by the photoreaction of [1.1.1]propellane and 2,3-butanedione is shown in the table below.

### [Comparative Example 1]

1,3-diacetyl BCP was obtained in the same manner as in Example 1, except that, in Example 1, THF was not blended in the reaction substrate solution. The yield of 1,3-diacetyl BCP obtained by the photoreaction of [1.1.1]propellane and 2,3-butanedione is shown in the table below.

### [Comparative Example 2]

1,3-diacetyl BCP was obtained in the same manner as in Example 5, except that, in Example 5, THF was not blended in the reaction substrate solution. The yield of 1,3-diacetyl BCP obtained by the photoreaction of [1.1.1]propellane and 2,3-butanedione is shown in the table below.

### [Comparative Example 3]

1,3-diacetyl BCP was obtained in the same manner as in Example 1, except that, in Example 1, ethyl acetate (EtOAc) was blended in the reaction substrate solution instead of THF. The yield of 1,3-diacetyl BCP obtained by the photoreaction of [1.1.1]propellane and 2,3-butanedione is shown in the table below.

### [Comparative Example 4]

1,3-diacetyl BCP was obtained in the same manner as in Comparative Example 3, except that, in Comparative Example 3, the ethyl acetate concentration in the reaction substrate solution was changed as shown in the table below. The yield of 1,3-diacetyl BCP obtained by the photoreaction of [1.1.1]propellane and 2,3-butanedione is shown in the table below.

### [Comparative Example 5]

1,3-diacetyl BCP was obtained in the same manner as in Example 1, except that, in Example 1, toluene was blended in the reaction substrate solution instead of THF. The yield of 1,3-diacetyl BCP obtained by the photoreaction of [1.1.1]propellane and 2,3-butanedione is shown in the table below.

### [Comparative Example 6]

1,3-diacetyl BCP was obtained in the same manner as in Comparative Example 5, except that, in Comparative Example 5, the toluene concentration in the reaction substrate solution was changed as shown in the table below. The yield of 1,3-diacetyl BCP obtained by the photoreaction of [1.1.1]propellane and 2,3-butanedione is shown in the table below.

### [Comparative Example 7]

1,3-diacetyl BCP was obtained in the same manner as in Example 1, except that, in Example 1, acetonitrile was blended in the reaction substrate solution instead of THF. The yield of 1,3-diacetyl BCP obtained by the photoreaction of [1.1.1]propellane and 2,3-butanedione is shown in the table below.

### [Comparative Example 8]

1,3-diacetyl BCP was obtained in the same manner as in Comparative Example 7, except that, in Comparative Example 7, the acetonitrile concentration in the reaction substrate solution was changed as shown in the table below. The yield of 1,3-diacetyl BCP obtained by the photoreaction of [1.1.1]propellane and 2,3-butanedione is shown in the table below.

### [Comparative Example 9]

1,3-diacetyl BCP was obtained in the same manner as in Example 1, except that, in Example 1, acetone was blended in the reaction substrate solution instead of THF. The yield of 1,3-diacetyl BCP obtained by the photoreaction of [1.1.1]propellane and 2,3-butanedione is shown in the table below.

### [Comparative Example 10]

1,3-diacetyl BCP was obtained in the same manner as in Comparative Example 9, except that, in Comparative Example 9, the acetone concentration in the reaction substrate solution was changed as shown in the table below. The yield of 1,3-diacetyl BCP obtained by the photoreaction of [1.1.1]propellane and 2,3-butanedione is shown in the table below.

### [Comparative Example 11]

1,3-diacetyl BCP was obtained in the same manner as in Comparative Example 2, except that, in Comparative Example 2, as the light source provided in the light source cooling pipe of the flow-type reaction system, an LED (100 W) lamp having a wavelength of 405 nm was provided instead of the LED (100 W) lamp having a wavelength of 385 nm. The yield of 1,3-diacetyl BCP obtained by the photoreaction of [1.1.1]propellane and 2,3-butanedione is shown in the table below.

### [Comparative Example 12]

1,3-diacetyl BCP was obtained in the same manner as in Comparative Example 2, except that, in Comparative Example 2, as the light source provided in the light source cooling pipe of the flow-type reaction system, an LED (100 W) lamp having a wavelength of 365 nm was provided instead of the LED (100 W) lamp having a wavelength of 385 nm. The yield of 1,3-diacetyl BCP obtained by the photoreaction of [1.1.1]propellane and 2,3-butanedione is shown in the table below.

**[Table 1]**

| | Solvent type | Reaction promoter | | Wavelength of light source (nm) | Yield (%) |
|---|---|---|---|---|---|
| | | Type | Concentration in reaction substrate solution (% by mass) | | |
| Example 1 | CPME/n-hexane | THF | 6.5 | 385 | 57 |
| Example 2 | CPME/n-hexane | THF | 13.4 | 385 | 87 |
| Example 3 | CPME/n-hexane | THF | 20.6 | 385 | 94 |
| Example 4 | CPME/n-hexane | THF | 34.1 | 385 | 96 |
| Example 5 | MTBE/n-hexane | THF | 6.5 | 385 | 58 |
| Example 6 | MTBE/n-hexane | THF | 13.4 | 385 | 85 |
| Example 7 | MTBE/n-hexane | THF | 20.6 | 385 | 93 |
| Example 8 | MTBE/n-hexane | THF | 34.1 | 385 | 96 |
| Example 9 | CPME/n-hexane | 2-methyl THF | 6.5 | 385 | 56 |
| Example 10 | CPME/n-hexane | 2-methyl THF | 13.4 | 385 | 83 |
| Example 11 | CPME/n-hexane | 2-methyl THF | 20.6 | 385 | 91 |
| Example 12 | CPME/n-hexane | 2-methyl THF | 34.1 | 385 | 94 |
| Example 13 | MTBE/n-hexane | 2-methyl THF | 6.5 | 385 | 55 |
| Example 14 | MTBE/n-hexane | 2-methyl THF | 13.4 | 385 | 84 |
| Example 15 | MTBE/n-hexane | 2-methyl THF | 20.6 | 385 | 93 |
| Example 16 | MTBE/n-hexane | 2-methyl THF | 34.1 | 385 | 97 |
| Example 17 | CPME/n-hexane | THF | 6.5 | 405 | 60 |
| Example 18 | CPME/n-hexane | THF | 34.1 | 405 | 95 |
| Example 19 | MTBE/n-hexane | THF | 6.5 | 405 | 61 |
| Example 20 | MTBE/n-hexane | THF | 34.1 | 405 | 97 |
| Example 21 | CPME/n-hexane | THF | 6.5 | 365 | 51 |
| Example 22 | CPME/n-hexane | THF | 34.1 | 365 | 94 |
| Example 23 | MTBE/n-hexane | THF | 6.5 | 365 | 52 |
| Example 24 | MTBE/n-hexane | THF | 34.1 | 365 | 95 |
| Comparative Example 1 | CPME/n-hexane | - | - | 385 | 10 |
| Comparative Example 2 | MTBE/n-hexane | - | - | 385 | 13 |
| Comparative Example 3 | CPME/n-hexane | EtOAc | 6.5 | 385 | 15 |
| Comparative Example 4 | CPME/n-hexane | EtOAc | 34.1 | 385 | 21 |
| Comparative Example 5 | CPME/n-hexane | Toluene | 6.5 | 385 | 14 |
| Comparative Example 6 | CPME/n-hexane | Toluene | 34.1 | 385 | 22 |
| Comparative Example 7 | CPME/n-hexane | Acetonitrile | 6.5 | 385 | 18 |
| Comparative Example 8 | CPME/n-hexane | Acetonitrile | 34.1 | 385 | 25 |
| Comparative Example 9 | CPME/n-hexane | Acetone | 6.5 | 385 | 20 |
| Comparative Example 10 | CPME/n-hexane | Acetone | 34.1 | 385 | 28 |
| Comparative Example 11 | MTBE/n-hexane | - | - | 405 | 18 |
| Comparative Example 12 | MTBE/n-hexane | - | - | 365 | 10 |

As shown in the above table, in a case where the tetrahydrofuran compound as cyclic ether was blended in the reaction substrate solution, as compared with a case where the tetrahydrofuran compound was not blended, it was found that the yield was dramatically improved (comparison of Comparative Example 1 and Examples 1 to 4 and 9 to 12, comparison of Comparative Example 2 and Examples 5 to 8 and 13 to 16, comparison of Comparative Example 11 and Examples 19 and 20, and comparison of Comparative Example 12 and Examples 23 and 24).

In addition, in a case where a compound other than the cyclic ether compound was tested as the reaction promoter, no effect of improving the yield was observed (Comparative Examples 3 to 10). From the results, it was found that the cyclic ether compound exhibited an excellent action as a reaction promoter in the photoreaction according to the present invention.

The present application claims the priority of JP2021-148842 filed in Japan on September 13, 2021.

### Explanation of References

1: reaction substrate solution (syringe pump)
2: constant-temperature tank
3: SUS tube
4: PFA tube
5: light source cooling pipe

## Claims

1. A method for producing 1,3-disubstituted bicyclo[1.1.1]pentane, the method comprising:
subjecting [1.1.1]propellane and a 1,2-diketone compound to a photoreaction in a solvent including an acyclic ether solvent having 5 or more carbon atoms to obtain 1,3-disubstituted bicyclo[1.1.1]pentane,
wherein a reaction solution coexists with a cyclic ether compound,
wherein the 1,2-diketone compound is a compound having a "*-C(=O)-C(=O)-*" structure, wherein * represents a linking site;
wherein the content of the cyclic ether compound in the reaction solution is 10% to 50% by mass.

2. The method for producing 1,3-disubstituted bicyclo[1.1.1]pentane according to claim 1,
wherein the acyclic ether solvent has 5 to 10 carbon atoms.

3. The method for producing 1,3-disubstituted bicyclo[1.1.1]pentane according to claim 1 or 2,
wherein the acyclic ether solvent has 5 or 6 carbon atoms.

4. The method for producing 1,3-disubstituted bicyclo[1.1.1]pentane according to any one of claims 1 to 3,
wherein the 1,2-diketone compound is a diacetyl compound or a glyoxylic acid compound.

5. The method for producing 1,3-disubstituted bicyclo[1.1.1]pentane according to any one of claims 1 to 4,
wherein the cyclic ether compound is a tetrahydrofuran compound.

6. The method for producing 1,3-disubstituted bicyclo[1.1.1]pentane according to any one of claims 1 to 5,
wherein the photoreaction is carried out by irradiation with light having a wavelength of 200 to 600 nm.

7. The method for producing 1,3-disubstituted bicyclo[1.1.1]pentane according to any one of claims 1 to 6,
wherein a reaction temperature of the photoreaction is set to -50°C to 50°C.

## Patentansprüche

1. Verfahren zum Produzieren von 1,3-disubstituiertem Bicyclo[1.1.1]pentan, wobei das Verfahren umfasst:
Unterziehen von [1.1.1]Propellane und einer 1,2-Diketonverbindung einer Photoreaktion in einem Lösungsmittel, das ein acyclisches Etherlösungsmittel mit 5 oder mehr Kohlenstoffatomen enthält, um 1,3-disubstituiertes Bicyclo[1.1.1]pentan zu erhalten,
wobei eine Reaktionslösung mit einer cyclischen Etherverbindung koexistiert,
wobei die 1,2-Diketonverbindung eine Verbindung mit einer "*-C(=O)-C(=O)-*"-Struktur ist,
wobei * eine Verknüpfungsstelle darstellt,
wobei der Gehalt der cyclischen Etherverbindung in der Reaktionslösung 10 Massen-% bis 50 Massen-% beträgt.

2. Verfahren zum Produzieren von 1,3-disubstituiertem Bicyclo[1.1.1]pentan nach Anspruch 1,
wobei das acyclische Etherlösungsmittel 5 bis 10 Kohlenstoffatome aufweist.

3. Verfahren zum Produzieren von 1,3-disubstituiertem Bicyclo[1.1.1]pentan nach Anspruch 1 oder 2,
wobei das acyclische Etherlösungsmittel 5 oder 6 Kohlenstoffatome aufweist.

4. Verfahren zum Produzieren von 1,3-disubstituiertem Bicyclo[1.1.1]pentan nach einem der Ansprüche 1 bis 3,
wobei die 1,2-Diketonverbindung eine Diacetylverbindung oder eine Glyoxylsäureverbindung ist.

5. Verfahren zum Produzieren von 1,3-disubstituiertem Bicyclo[1.1.1]pentan nach einem der Ansprüche 1 bis 4,
wobei die cyclische Etherverbindung eine Tetrahydrofuranverbindung ist.

6. Verfahren zum Produzieren von 1,3-disubstituiertem Bicyclo[1.1.1]pentan nach einem der Ansprüche 1 bis 5,
wobei die Photoreaktion durch Bestrahlung mit Licht mit einer Wellenlänge von 200 bis 600 nm durchgeführt wird.

7. Verfahren zum Produzieren von 1,3-disubstituiertem Bicyclo[1.1.1]pentan nach einem der Ansprüche 1 bis 6,
wobei eine Reaktionstemperatur der Photoreaktion auf -50 °C bis 50 °C eingestellt ist.

## Revendications

1. Procédé de production de bicyclo[1.1.1]pentane 1,3-disubstitué, le procédé comprenant :
soumettre [1.1.1]propellane et un composé 1,2-dicétone à une photoréaction dans un solvant incluant un solvant éther acyclique ayant 5 atomes de carbone ou plus pour obtenir bicyclo[1.1.1]pentane 1,3-disubstitué,
dans lequel une solution réactionnelle coexiste avec un composé éther cyclique,
dans lequel le composé 1,2-dicétone est un composé ayant une structure « *-C(=O)-C(=O)-* »,
dans lequel * représente un site de liaison,
dans lequel la teneur en composé éther cyclique dans la solution réactionnelle est de 10 % à 50 % en masse.

2. Procédé de production de bicyclo[1.1.1]pentane 1,3-disubstitué selon la revendication 1,
dans lequel le solvant éther acyclique a de 5 à 10 atomes de carbone.

3. Procédé de production de bicyclo[1.1.1]pentane 1,3-disubstitué selon la revendication 1 ou la revendication 2,
dans lequel le solvant éther acyclique a 5 ou 6 atomes de carbone.

4. Procédé de production de bicyclo[1.1.1]pentane 1,3-disubstitué selon l'une quelconque des revendications 1 à 3,
dans lequel le composé 1,2-dicétone est un composé diacétyle ou un composé d'acide glyoxylique.

5. Procédé de production de bicyclo[1.1.1]pentane 1,3-disubstitué selon l'une quelconque des revendications 1 à 4,
dans lequel le composé éther cyclique est un composé tétrahydrofuran.

6. Procédé de production de bicyclo[1.1.1]pentane 1,3-disubstitué selon l'une quelconque des revendications 1 à 5,
dans lequel la photoréaction est réalisée par irradiation avec une lumière ayant une longueur d'onde de 200 à 600 nm.

7. Procédé de production de bicyclo[1.1.1]pentane 1,3-disubstitué selon l'une quelconque des revendications 1 à 6,
dans lequel une température de réaction de la photoréaction est réglée entre -50 °C et 50 °C.
